# EUROPEAN PATENT SPECIFICATION

(11) **EP 2 902 019 B1**
(45) Date of publication and mention of the grant of the patent: **04.10.2017**
(21) Application number: 15153102.7
(22) Date of filing: 01.08.2011
(51) Int. Cl.: A61K 31/132, A61K 8/41, A61Q 11/00, A61K 8/73, A61Q 19/00, A61Q 19/08, A61Q 19/10, A61K 8/04, A61K 31/724, A61P 1/02, A61K 9/00, A61K 9/02, A61K 9/06, A61K 9/10, A61K 9/14, A61K 47/08, A61K 47/38, A61K 47/40, A61K 47/10, A61K 47/18

(54) **Compositions comprising inclusion complexes of cyclodextrins with spermidine for use in oral care**
Zusammensetzungen mit Einschlusskomplexen von Cyclodextrinen mit Spermidin zur Verwendung in der Mundpflege
Compositions comprenant des complexes d'inclusion de cyclodextrines avec de la spermidine destinées à être utilisées pour des soins buccaux

(30) Priority: 04.08.2010 IT MI20101491; 14.12.2010 IT MI20102277; 16.12.2010 IT MI20102308
(43) Date of publication of application: 05.08.2015
(62) Divisional of application: 11814166.2
(73) Proprietor: PIERREL PHARMA S.R.L., 81043 Capua (CE) (IT)
(72) Inventor: GHISALBERTI, Carlo, 04542-080 Sao Paulo (BR)
(74) Representative: Ripamonti, Enrico

(56) References cited:
- OGURI S ET AL: "Development of a simple high-performance capillary electrophoretic method with on-line mode in capillary derivatization for the determination of spermidine.", ELECTROPHORESIS NOV 1998, vol. 19, no. 16-17, November 1998 (1998-11), pages 2986-2990, XP002718004, ISSN: 0173-0835
- MALE K B ET AL: "Derivatization, stabilization and detection of biogenic amines by cyclodextrin-modified capillary electrophoresis-laser-induced fluorescence detection", JOURNAL OF CHROMATOGRAPHY, ELSEVIER SCIENCE PUBLISHERS B.V, NL, vol. 926, no. 2, 17 August 2001 (2001-08-17), pages 309-317, XP004298628, ISSN: 0021-9673, DOI: 10.1016/S0021-9673(01)01056-1
- W. SAENGER, T. STEINER: "Cyclodextrin Inclusion Complexes: Host-Guest Interactions and Hydrogen-BOnding Networks", ACTA CRYST., vol. A54, 1998, XP002718005, Great Britain ISSN: 0108-7673
- STABELLINI G ET AL: "Effects of spermine and spermidine on glycosaminoglyan metabolism in primary cultures of chick embryo fibroblasts", MEDICAL SCIENCE RESEARCH, ELSEVIER APPLIED SCIENCE, BARKING, GB, vol. 17, no. 21, 1 January 1989 (1989-01-01), pages 915-916, XP009098831, ISSN: 0269-8951

## Description

### FIELD OF THE INVENTION

The invention refers to compositions comprising inclusion complexes formed by cyclodextrins and spermidine or salt thereof having high proliferative activities for use for use in the treatment, maintenance or repair of periodontum and oral tissues.

### BACKGROUND

Spermidine belongs to the group of the biogenic polyamines (PA). These ubiquitary, metabolic substances play a complex role in cell regulation such as differentiation, proliferation and apoptosis according to the environment conditions (Flamigni F et al. Amino Acids. 2007;33(2):197-202; Seiler & Raul. J Cell Mol Med. 2005;9(3):623-42; Ray RM et al. Am J Physiol Cell Physiol. 2000; 278(3):C480-9; Hughes A et al. Biochem J. 2003;374(Pt 2):481-8; Schipper RG et al. Semin Cancer Biol. 2000;10(1):55-68).

Indeed, spermidine has a bimodal pattern primarily driven by its local level, meaning that it acts either as growth factor or inhibitor, as in US 4,507,321 (New York Univ.) wherein the ability to stimulate or depress epithelial cell growth correlates with low and high concentrations, respectively.

The only known medicinal applications of PA are the anti-alopecia products marketed as Bioscalin® by Giuliani SpA (Milan, Italy), whose active ingredient is spermidine HCl, herein branded as Biogenina or Cronobiogenina therein.

WO2010017328 discloses cyclodextrins covalently conjugated to a variety cationic arms, including polyamines. These newly modified cyclodextrins have been synthesized to deliver anionic charged molecules and facilitate the cellular uptake of double-stranded or hairpin nucleic acids.

Our concomitant divisional PCT application discloses the supramulecular complexes formed by polyanionic polymers and spermidine having anionic equivalents to cationic equivalents ratio ≥ 10:1, which own high potency in fibroblast proliferation.

Since many therapeutic/aesthetic issues are in need of tissue regeneration and repair, any advanced delivery form of spermidine optimizing its mitotic action is of high value.

### SUMMARY

The invention provides compositions comprising inclusion complexes formed by cyclodextrins and spermidine or salt thereof.

In one aspect, the inclusion complex has a stoichiometry of cyclodextrin to spermidine from 1:1 to 10:1 mol/mol, inclusive and more preferably 2:1 mol/mol.

In another aspect, the invention provides compositions for oral care comprising inclusion complex of spermidine into cyclodextrin, wherein spermidine is present in non-covalent, ring-closed form.

In still another aspect, the invention provides medicinal/cosmetic compositions for use in the oral connective tissue repair comprising an inclusion complex cyclodextrins-spermidine in amount from 1% to 0.001 % w/w, more preferably from 0.1 % to 0.01 % w/w of the composition in the presence of physiologically acceptable carriers and ingredients.

The invention further relates to compositions and methods for the repair and maintenance of oral connective tissue which may be prompted by the controlled release of spermidine from the cyclodextrin complexes.

These and other aspects will be further illustrated in the foregoing disclosure.

### BRIEF DESCRIPTION OF THE DRAWINGS

FIG. 1 and 2 are the FT-IR spectra of HPβCD-spermidine.HCl 2:1 mol:mol complex and the reference HPβCD, respectively.
FIG. 3 and 4 are the FT-IR spectra of αCD-spermidine.HCl 2:1 mol:mol complex and reference αCD, respectively.
FIG. 5 and 6 are the FT-IR spectra of RMβCD-spermidine.HCl 2:1 mol:mol complex and the reference RMβCD, respectively.

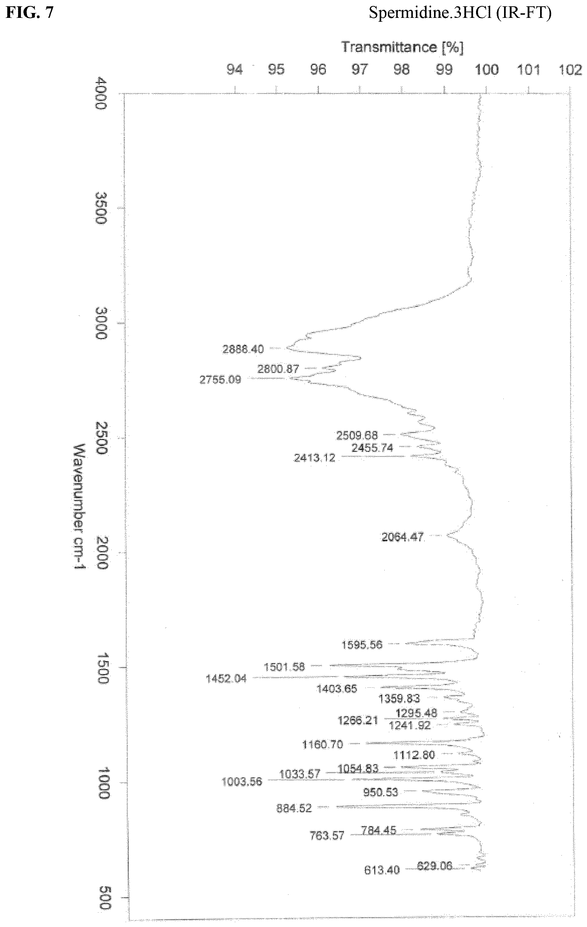

FIG. 7 is the FT-IR spectrum of spermidine.3HCl 1:1.
FIG. 8 shows the % fibroblast increase after treatment with αCD-spermidine.HCl 2:1 mol/mol at serial dilutions over control (untreated cells).
FIG. 9 shows the % fibroblast increase after treatment with spermidine.3HCl at serial dilutions over control (untreated cells).
FIG. 10 plots the potency ratio of αCD-spermidine.HCl 2:1 vs spermidine 3HCl alone expressed as the respective % : % fibroblast increase.

### DETAILED DESCRIPTION

The invention refers to compositions comprising cyclodextrin inclusion complexes of spermidine for use in oral care.

Formulations comprising such complexes was found to release spermidine in controlled fashion, wherein it may then reach the target side at pmolar concentration, thereby acting as a stimulatory/growth agent for the repair and maintenance of the connective tissue.

The expressions "inclusion complex" or "supramolecular complex" or simply "complex" could be considered as synonyms in the context of the present invention, and encompass the physical combination of one or more cyclodextrin with spermidine, or salt thereof.

More specifically, the expression "inclusion complex" is used according the meaning of a host-guest chemistry, wherein the cyclodextrin(s) hosts within their internal cavity spermidine, hence with no chemical bond (of covalent type) between the counterparts.

In an embodiment, the spermidine used in the present invention is substance of formula NH₂(CH₂)₃NH(CH₂)₄NH₂ (C₇H₁₉N₃) *alias* N-(3-aminopropyl)-1,4-butanediamine, herein as free base or as salt thereof. Preferred spermidine is of synthetic origin; even more preferably has a purity of at least 99% on dry basis.

Exemplary such salts are spermidine monohydrochloride, spermidine dihydrochloride, and spermidine trihydrochloride.

For the inventive purpose, cyclodextrins of various grades that may be used include, but not limited to: α-cyclodextrin ("αCD"), β-cyclodextrin ("βCD"), γ-cyclodextrin ("γCD"), and the cyclodextrins derivatives.

A cyclodextrin derivative is understood to be a cyclodextrin or mixtures thereof wherein the hydrogen(s) of a part or all of the hydroxyl groups at positions 2-, 3- and 6- of glucose is(are) replaced by (an)other functional group(s), such as a dihydroxyalkyl group, a saccharide residue, a hydroxyalkyl group, a sulfonate group, a sulfoalkyl group, an alkyl group, an alkanoyl group, an acetyl group or a benzoyl group.

The cyclodextrin or its derivatives used in this invention may be commercially available or may be synthesised by a method known per se. Examples of cyclodextrin and its derivatives comprise natural cyclodextrins (α, β, or γ), hydroxypropyl cyclodextrins ("HPβCD"), randomly methylated β-cyclodextrin ("RMβCD"), carboxymethyl cyclodextrins, sulfobutylcyclodextrins, aminocyclodextrin, dimethyl cyclodextrin, cyclodextrin phosphate, hydroxyethylcyclodextrin, acetyl-cyclodextrin, ethylcyclodextrins, trimethylcyclodextrins, carboxyethylcyclodextrin, glucosylcyclodextrin, maltosyl cyclodextrins, butyl-cyclodextrins, sulfated cyclodextrins, N,N-diethylaminoethyl cyclodextrin, tert-butylsilylcyclodextrins, silyl[(6-O-tert-butyldimethyl)-2,3-di-O-acetyl)-cyclodextrins, succinyl-(2-hydroxypropyl)-cyclodextrins, succinyl-cyclodextrins, sulfopropyl-cyclodextrins, and polycyclodextrins.

The mean degree of substitution (DS) refers to the mean number of hydroxyls substituted per unit of CD, whereas the degree of molar substitution refers to the number of hydroxyl groups per unit of anhydroglucose. In this invention, the cyclodextrins used exhibit a mean degree of substitution from 4.2 to 7, although CD with a DS beyond that may be applied.

Commercial sources of cyclodextrins include ISP Corp. (Wayne, New Jersey, USA); Cargill, Inc. (Wayzata, Minn. USA); Roquette Freres (Lestrem, France); Aldrich Chemical Company (Milwaukee, Wis. USA);. and Wacker Chemicals (New Canaan, Connecticut. USA). Other useful cyclodextrins are described by Uekama K (Chem. Pharm. Bull. 2004; 52(8) 900-15); or can be synthesized by processes known in the art for the synthesis of cyclodextrins and their cyclodextrins derivatives.

In a particular embodiment of this invention, the cyclodextrin is α-cyclodextrin ("αCD"), hydroxypropyl-β-cyclodextrin ("HPβCD"), randomly methylated β-cyclodextrin ("RMβCD"), and β-cyclodextrin ("βCD"), or mixtures thereof.

According to the invention, spermidine has been found to be included in the cavity of the cyclodextrins forming a molecular inclusion complexes suitable for its controlled release.

The expression "controlled release" as used herein includes "sustained release", "slow release" and, more generally, the controlled release of spermidine from the inclusion complex so to optimize its activity as growth factor.

The formation of inclusion complex is evident from the Fourier-transform infrared ("FT-IR") spectra, the differential scanning calorimetry ("DSC") curves, or the X-ray powder diffraction ("XRPD") patterns. Note that attached FT-IR spectra have been obtained from dried samples compressed into KBr pellets.

According to the present invention, molar ratios of cyclodextrins to spermidine within the inclusion complexes may range from about 1:1 to about 1:10 mol/mol, or from about 1:1 to about 1:4 mol/mol, inclusive and more preferably 2:1 mol/mol.

The inclusion complexes may be prepared according to methods known in the art, including dry and wet methods, and combination thereof. The methods of preparation include: reaction in solvent, wherein cyclodextrins and spermidine are solubilized in solvent(s), generally water; granulation, wherein wet or dry spermidine is compounded to cyclodextrins; and spraying, wherein a spermidine solution is sprayed on dry cyclodextrin.

In one embodiment, the inclusion complexes obtained as solution are used as such, provided that the solvent is compatible with the purposes of the composition.

In another embodiment, the inclusion complexes are isolated in a substantially dry form, for example the solvent is removed in a warmed air stream, or by liophylization, or at moderate temperature under vacuum, or by spray-drying, and combination thereof.

A method to obtain the inclusion complex of invention in solid state is precipitation, wherein the complex is precipitated from a concentrated solution, e.g. aqueous, obtained by stirring the complex components at, e.g., 50-70°C, followed by cooling and filtration.

In accordance with an aspect of the present invention, there are provided compositions and methods to regenerate oral connective tissues by eliciting/enhancing cell proliferation.

In several differentiated embodiments of present invention, the compositions comprising said inclusion complexes are administered to improve the trophism of oral senescent tissues or to repair oral damaged tissues of oral mucosa, i.e. in stomatology, to treat stomatitis, aphtous ulcer, dry mouth and/or Sjogren's syndrome, as well as in gingiva and periodontum, i.e. in periodontology, to treat gingivitis/periodontitis.

The inventive compositions comprising inclusion complexes for use according to the invention, have a high regenerative activity on fibroblasts, and progenitors and differentiated cells thereof, hence are suitable for the preparation of medicinal and cosmetic composition with regenerative/reparative purposes.

The amount of the inclusion complex cyclodextrin.spermidine may vary from about 50% to about 0.0005% w/w, preferably from about 5% to about 0.005% w/w, even more preferably from about 0.5% to about 0.05% w/w of the composition.

Low dosage levels are recommended in leave-in compositions, wherein a long period of time to allow the release of a controlled/sustained amount of spermidine onto target tissue.

On the other side, the upper dosage levels are recommended in rinse-off or short-time contact to provide a sufficient amount of spermidine which may elicit a regenerative action onto the connective and related tissues.

The composition of invention may be produced according to known techniques with physiologically acceptable ingredients and carriers in order to afford the better benefit/risk profile, e.g. those listed in INCI-CTFA Annex 93/35/ECC and, or in Pharmacopoeias.

The compositions for oral care may be formulated in many ways, e.g., as per ADA/PDR: Guide to Dental Therapeutics, 4th Ed. by Physicians' such as mouthwash, oral solution, spray, gel on film, dentifrices, tooth powder, dental tablets, cream and gels, chewing gum, chewable tablets and lozenges, gel, film, gel-on-film, granules, paste, spreadable powders, etc. for application on oral cavity directly or by an external device.

### EXAMPLES

The following example is illustrative of an embodiment of an inclusion complex suitable for the oral use according to the invention. The inclusion complex *per se* is not falling within the scope of the present invention.

### EXAMPLE 1 - Inclusion complex HPβCD-spermidineHCl 2:1 mol/mol

2.80 g of hydroxypropyl-β-cyclodextrin (Kleptose HPB Oral; Roquette SA, Lestem, France) were dissolved in 4.6 ml of water at ambient temperature. 255 mg of spermidine 3HCl were then added, followed by 0.2 ml of NaOH 1N under slow stirring. A slight alkaline pH (∼8), transparent solution of the complex was obtained.

Portions have been used as such in water-based compositions. A partion was vacuum dried to afford dried complex (H₂O < 5%) for applicative purposes and characterization.

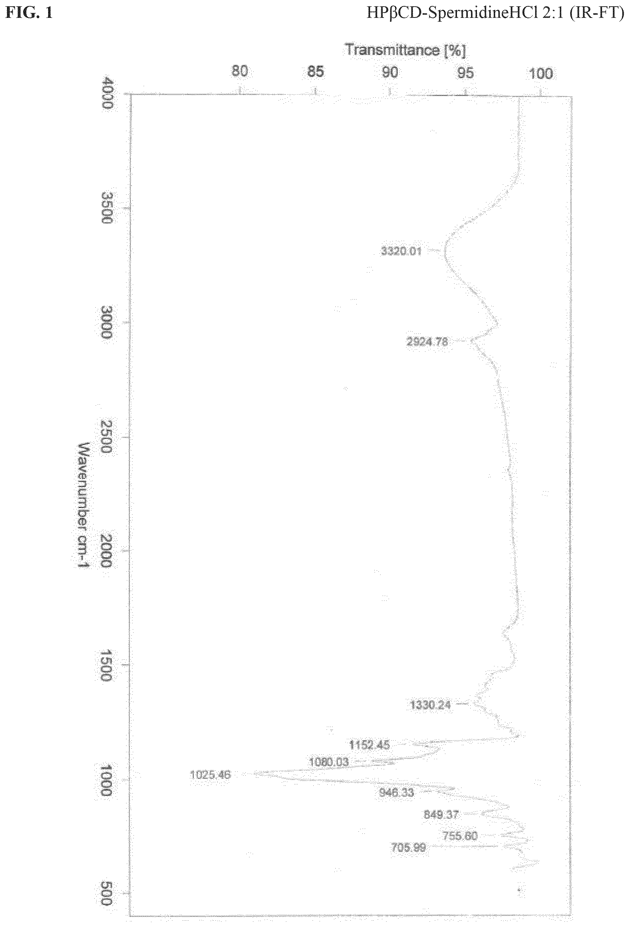

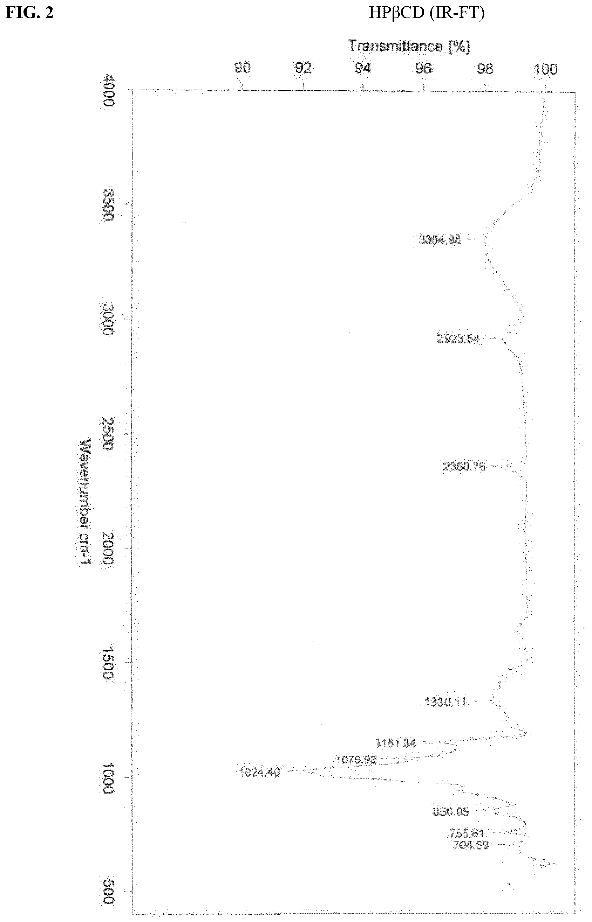

FIGs 1 and 2 are FT-IR spectra of HPβCD-SpdHCl 2:1 mol/mol and the reference βCD. The following example is illustrative of an embodiment of an inclusion complex suitable for the oral use according to the invention. The inclusion complex *per se* is not falling within the scope of the present invention.

### EXAMPLE 2 - Inclusion complex αCD-spermidineHCl 2:1 mol/mol

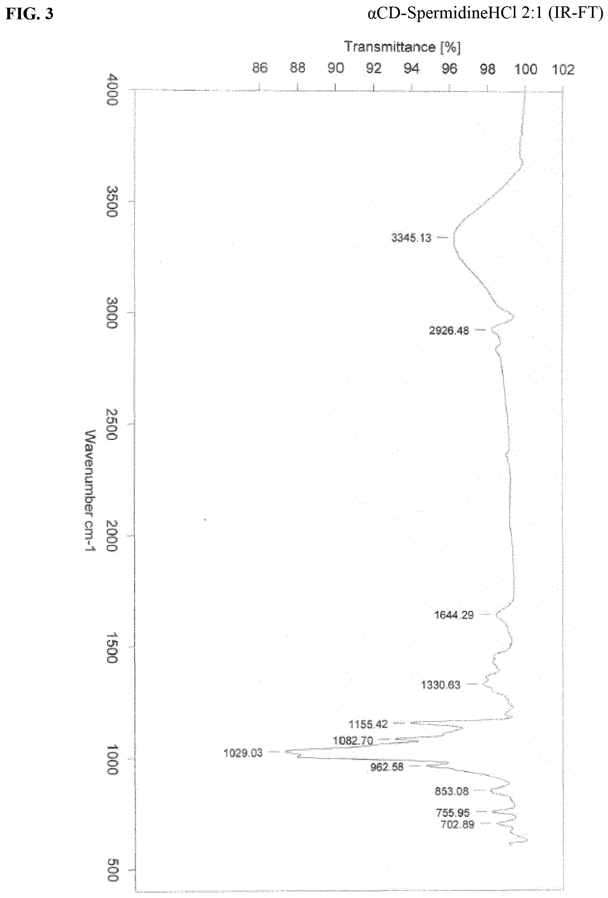

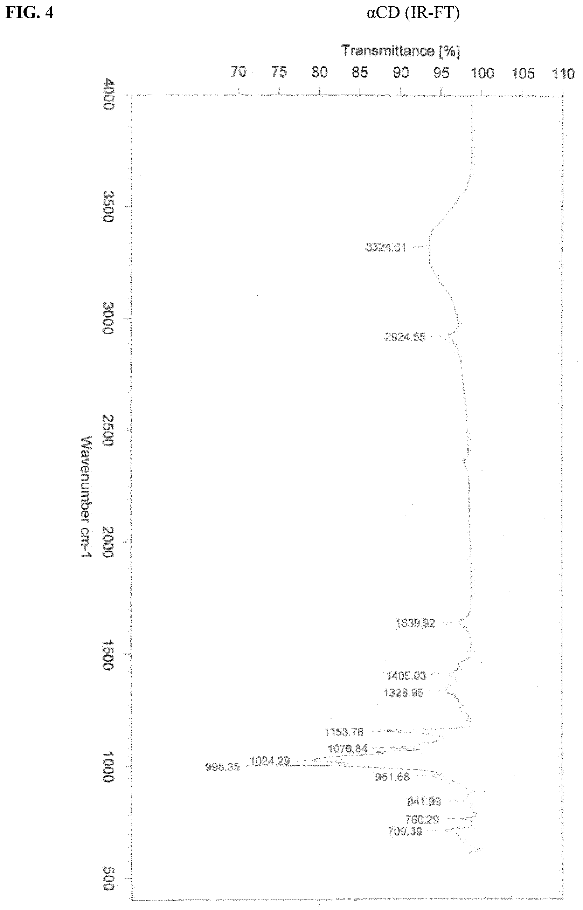

1.944 g of α-cyclodextrin (Cawamax W6: ISP; Wayne, NJ, USA) were dissolved in 15 ml of water. This solution was added with 1 mmole of spermidine 3HCl, then 0.2 ml of NaOH 1N under slow stirring. Similarly the product can be used as solution or in dried form, whose FT-IR spectrum is in FIG. 3; whilst FIG. 4 is the starting αCD.

The following example is illustrative of an embodiment of an inclusion complex suitable for the oral use according to the invention. The inclusion complex *per se* is not falling within the scope of the present invention.

### EXAMPLE 3 - Inclusion complex RMβCD-spermidineHCl 2:1 mol/mol

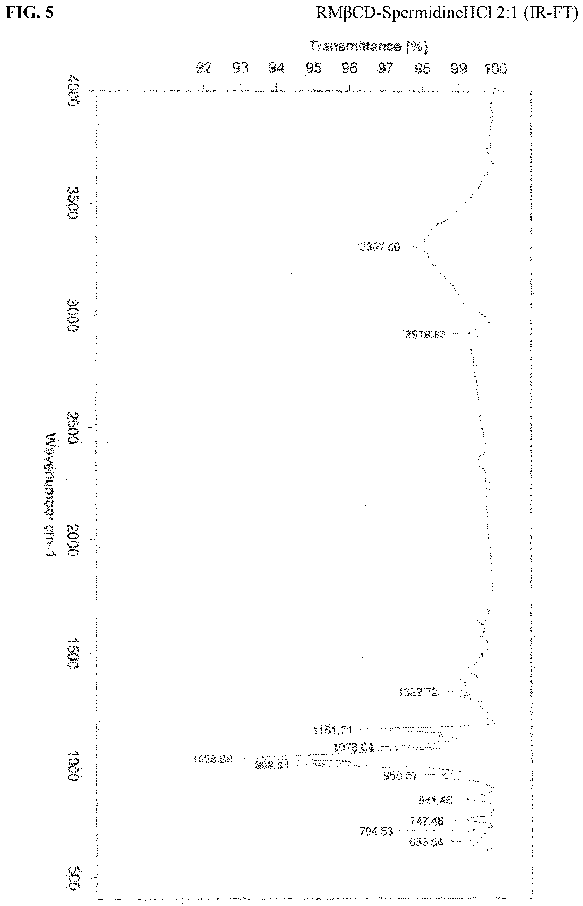

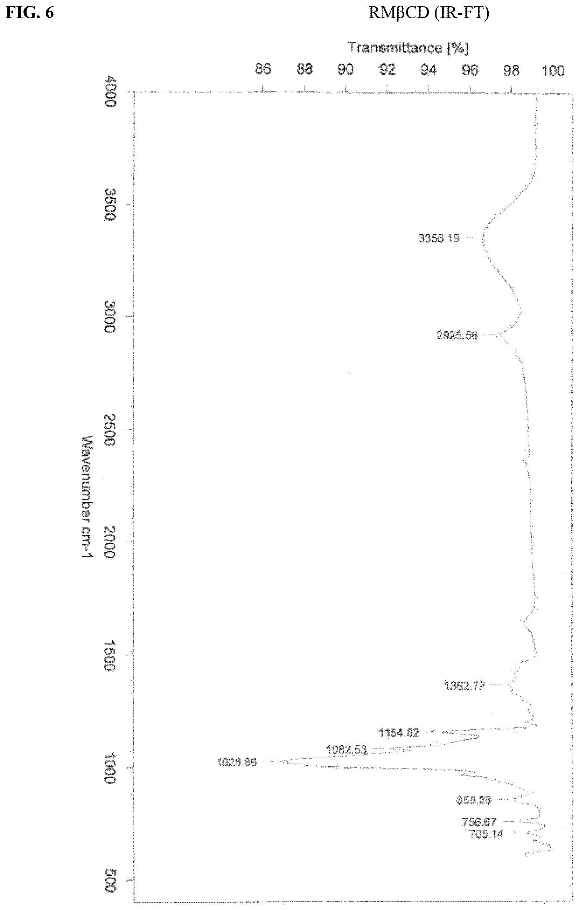

2,624 g of randomly methylated β-cyclodextrin (Kleptose Crysmeb; Roquette) were dissolved in 9 ml of water. This solution was added with 1 mmol of spermidine 3HCl and 0.2 ml of NaOH 1N under slow stirring. FT-IR spectra of the dry RMβCD-spermidineHCl 2:1 complex and pure RMβCD are the FIG. 5 and 6, respectively.

### EXAMPLE 4 - Fibroblasts proliferation by αCD-spermidineHCl 2:1 mol/mol

The inclusion complex αCD-SpdHCl 2:1 mol/mol as obtained in Example 2 was used to assess the ability to stimulate the proliferation of human fibroblasts (ATCC-CRL-2703).

Culture media containing tested samples at chosen concentrations were added to the wells containing cells in the G0 phase of cell cycle. Cells were exposed to each product for 24 and 48 hours (medium was replaced every 24 h) at 37°C and 5% CO₂. At the end of incubation period, MTT test was performed to evaluate cell viability and proliferating rate compared to untreated cells, each determination carried out in sextuplicate. After exposure, cells were washed with 200 ml of PBS, then 200 µl of MTT-medium were added and culture well incubated for 4 h at 37°C and 5% CO₂. MTT medium is then removed and 200 µl of MTT Solubilization Solution (10% Triton X-100 and 0.1 N HCl in dry isopropanol) were added. After shaking on rotatory plate for 20-30', absorbance was read at 570 nm; background reading at 690 nm. Results expressed as % cell *vs* control cells are in Table I.

**TABLE I**

| αCD-SpermidineHCl 2:1 (concentration) | % Proliferative increase | |
|---|---|---|
| | 24 h | 48 h |
| 1 mM | -0.84 | 8.48 |
| 100 µM | 18.69 | 24.48 |
| 10 µM | 48.65 | 45.68 |
| 1 µM | 61.62 | 62,87 |
| 100 nM | 83.00 | 80.65 |
| 10 nM | 42.59 | 84.30 |
| 1 nM | 27.44 | 43.97 |
| 0.1 nM | 11.11 | 5.85 |

The results are expressed as % increase in fibroblasts treated with inclusion complexes for 24h and 48 h versus untreated (control) cells.

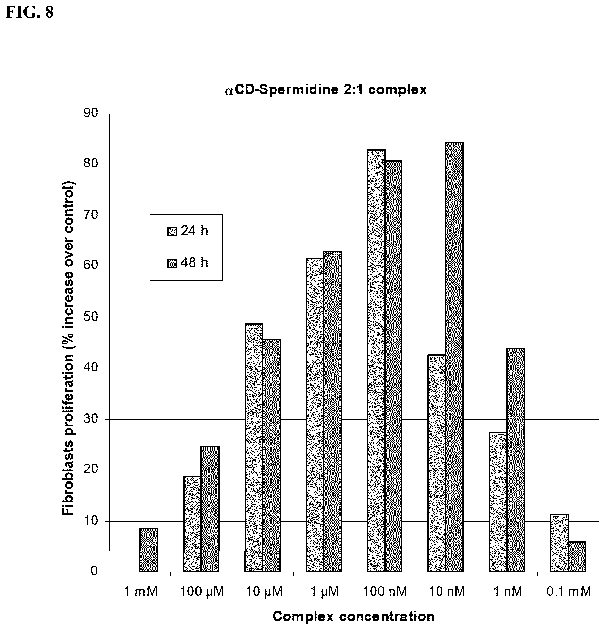

The plotted data (FIG. 8) show that the inventive complexes actually express their maximum potency within the micromolar range.

### COMPARATIVE EXAMPLE - Spd 3HCl vs αCD-SpdHCl 1:1 in fibroblast proliferation

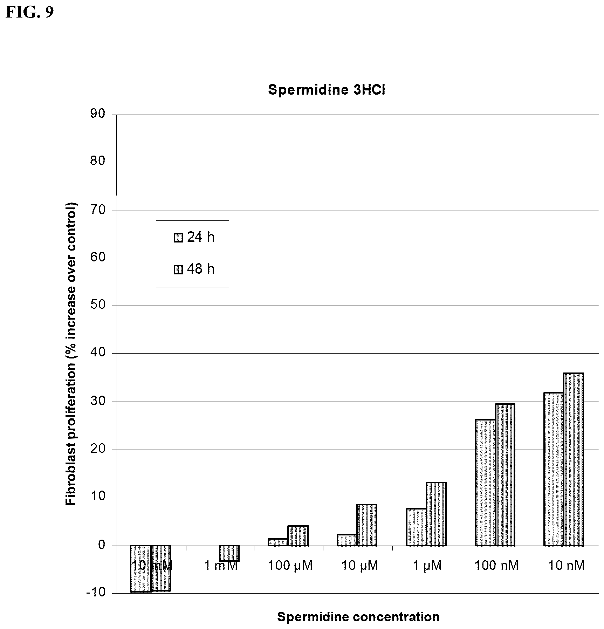

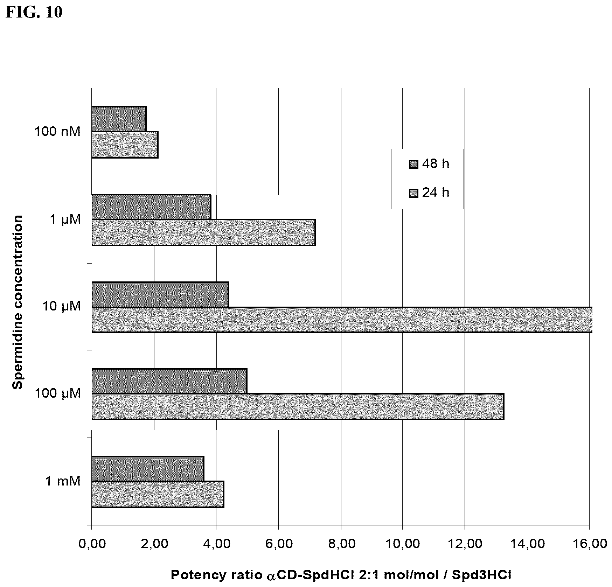

The test as described in Example 4 was carried out at with Spd 3HCl at serial dilutions with results shown in FIG. 9. Potency ratios expressed as % / % fibroblasts increase of αCD-Spd 2:1 / Spd3HCl at 24h and 4h are illustrated in Table II, and plotted in FIG. 10.

**TABLE II**

| Spermidine (equivalent) concentration | Proliferative ratio αCD-Spd 2:1 / Spd3HCl | |
|---|---|---|
| | 24 h | 48 h |
| 100 µM | 14.27 | 5.99 |
| 10 µM | 21.15 | 5.39 |
| 1 µM | 8.16 | 4.83 |
| 100 nM | 3.16 | 2.74 |

The data well illustrate the enhanced potency in fibroblast proliferation of the inclusion complexes over free spermidine, and their utility in regenerative/reparative purposes.

The following example is illustrative of an embodiment of a composition comprising an inclusion complex suitable for the oral use according to the invention.

### EXAMPLE 6 - Gel #1

A gel was prepared having ingredients as set forth in Table IV below.

**TABLE IV**

| Ingredient | Quantity (in 100 ml) |
|---|---|
| αCD-SpdHCl 2:1 mol/mol, 10% sol. | 0.20 ml |
| Poloxamer (Lutrol F127). | 18.0 g |
| Methylisothiazolinone, 0.9% sol. (MT) | 0.10 ml |
| Purified water | to 100 ml |

Poloxamer was dissolved in water, then the preservative Microcare® MT (Thor Personal Care) and the αCD-Spd aqueous solution were sequentially added to afford a viscous gel. The following example is illustrative of an embodiment of a composition comprising an inclusion complex suitable for the oral use according to the invention.

### EXAMPLE 7 - Gel #2

0.1 ml of αCD-SpdHCl 2:1 mol/mol 10% aqueous solution were suspended in Carbopol gel preserved with 0.01 % w/v methylisothiazolinone.

The following example is illustrative of an embodiment of a composition comprising an inclusion complex suitable for the oral use according to the invention.

### EXAMPLE 8 - Solution

A solution was prepared having ingredients as set forth in Table VII below.

**TABLE VII**

| Ingredient | Quantity (in 100 ml) |
|---|---|
| αCD-SpdHCl 2:1 mol/mol, 10% sol. | 0.10 ml |
| Methylisothiazolinone, 0.9% sol. (MT) | 0.10 ml |
| Hydroxyethyl cellulose | 0.30 g |
| Anethol | 0.40 g |
| Cremophor EL | 0.40 g |
| Sodium saccharinate | 0.05 g |
| Purified water | to 100 ml |

The following example is illustrative of an embodiment of a composition comprising an inclusion complex suitable for the oral use according to the invention.

### EXAMPLE 9 - Chewing gum

αCD-SpdHCl 2:1 mol/mol dry was mixed with gum base (Gum Base Co SpA, Lainate, Italy) cooled to -10° C in mill, for 20' at 20°C. Customary ingredients were incorporated, sieved at 50 mesh and extruded to afford soft tablets of ∼1.4 g each.

The above-described preferred embodiments and examples of the present invention are not intended to limit the scope of the invention. Modifications or alterations may be made therein without departing from the spirit and the scope of the invention as set forth in the appended claims.

## Claims

1. A composition comprising an inclusion complex of cyclodextrins and spermidine in ratios from 1:1 to 10:1 mol/mol, with no covalent bonds thereto, for use in oral care.

2. The composition for use according to claim 1, wherein the cyclodextrins to spermidine ratio is 2:1 mol/mol, where spermidine is a in ring-closed form.

3. The composition for use according to claim 1 wherein spermidine is present as a partial salt such as monohydrochloride or as free base.

4. The composition for use according to claim 1 wherein the cyclodextrin is selected from the group consisting of α-cyclodextrin, hydroxypropyl-β-cyclodextrin, randomly methylated β-cyclodextrin, and β-cyclodextrin.

5. The composition for use according to any of claims 1-4 in aqueous solution.

6. The composition for use according to any of claims 1-4 in a substantially dry form.

7. The composition for use according to any of claims 1-6, for use in the maintenance and repair of a damaged or senescent oral connective tissue selected from the group consisting of throat mucosae, oral mucosa, gingiva and periodontum.

8. The composition for use according to claim 7 for the treatment of stomatitis, aphtous ulcer and dry mouth.

9. The composition for use according to claim 7 for the treatment of gingivitis or periodontitis.

10. The composition for use according to any of claims 1-10, which comprises an inclusion complex of cyclodextrins and spermidine at ratios from 1:1 to 1:4 mol:mol in an amount from 5% to 0.005% w/w of a dosage form for topical, intramucosal or oral administration.

## Patentansprüche

1. Zusammensetzung mit Einschlusskomplex von Cyclodextrinen und Spermidin in einem Verhältnis von 1:1 bis 10:1 mol/mol, ohne kovalente Bindungen damit, zur Verwendung in der Mundpflege.

2. Zusammensetzung zur Verwendung nach Anspruch 1, worin das Cyclodextrin/Spermidin-Verhältnis 2:1 mol/mol beträgt, worin Spermidin eine ringgeschlossene Gestalt hat.

3. Zusammensetzung zur Verwendung nach Anspruch 1, worin Spermidin als Teilsalz, wie zum Beispiel Monohydrochlorid, oder als freie Base vorhanden ist.

4. Zusammensetzung zur Verwendung nach Anspruch 1, worin das Cyclodextrin aus der Gruppe bestehend aus α-Cyclodextrin, Hydroxypropyl-β-cyclodextrin, statistisch methyliertem β-Cyclodextrin, und β-Cyclodextrin gewählt wird.

5. Zusammensetzung zur Verwendung nach irgendeinem der Ansprüche 1-4 in wässriger Lösung.

6. Zusammensetzung zur Verwendung nach irgendeinem der Ansprüche 1-4 in einer wesentlich trockenen Form.

7. Zusammensetzung zur Verwendung nach irgendeinem der Ansprüche 1-6, zur Verwendung zur Pflege und Reparatur von einem beschädigten oder alternden mündlichen Bindegewebe, das aus der Gruppe bestehend aus Halsschleimhaut, Mundschleimhaut, Gingiva und Parodont gewählt wird.

8. Zusammensetzung zur Verwendung nach Anspruch 7 zur Behandlung von Stomatitis, aphthösen Geschwüren und Mundtrockenheit.

9. Zusammensetzung zur Verwendung nach Anspruch 7 zur Behandlung von Gingivitis oder Parodontitis.

10. Zusammensetzung zur Verwendung nach irgendeinem der Ansprüche 1-10, umfassend einen Einschlusskomplex von Cyclodextrinen und Spermidin in einem Verhältnis von 1:1 bis 1:4 mol:mol, in einer Menge von 5 % bis 0,005 % w/w einer Darreichungsform zur topischen, intramukosalen oder oralen Verabreichung.

## Revendications

1. Composition comprenant un complexe d'inclusion de cyclodextrines et de spermidine dans un rapport entre 1:1 et 10:1 mol/mol, sans liaisons covalentes avec elle, pour une utilisation dan des soins buccaux.

2. Composition pour l'utilisation selon la revendication 1, où le rapport entre cyclodextrines et spermidine est de 2:1 mol/mol, où la spermidine est en forme d'anneau fermé.

3. Composition pour l'utilisation selon la revendication 1, où la spermidine est présente comme sel partiel tel que monochlorhydrate ou comme base libre.

4. Composition pour l'utilisation selon la revendication 1, où la cyclodextrine est sélectionnée parmi le groupe de α-cyclodextrine, hydroxypropyl-β-cyclodextrine, β-cyclodextrine méthylée au hasard et β-cyclodextrine.

5. Composition pour l'utilisation selon l'une quelconque des revendications 1-4 dans une solution aqueuse.

6. Composition pour l'utilisation selon l'une quelconque des revendications 1-4 dans une forme substantiellement sèche.

7. Composition pour l'utilisation selon l'une quelconque des revendications 1-6, pour une utilisation dans l'entretien et la réparation d'un tissu conjonctif endommagé ou sénescent sélectionné parmi le groupe de la muqueuse de la gorge, de la muqueuse orale, de la gencive et du parodonte.

8. Composition pour l'utilisation selon la revendication 7 pour le traitement de la stomatite, l'ulcère aphteux et la bouche sèche.

9. Composition pour l'utilisation selon la revendication 7 pour le traitement de la gingivite ou de la parodontite.

10. Composition pour l'utilisation selon l'une quelconque des revendications 1-10, comprenant un complexe d'inclusion de cyclodextrines et de spermidine dans un rapport entre 1:1 et 1:4 mol:mol dans une quantité entre 5% et 0,005% p/p d'une forme de dosage pour administration par voie topique, intramuqueuse ou orale.
